# EUROPEAN PATENT APPLICATION

(11) **EP 2 562 151 A1**
(43) Date of publication of application: **27.02.2013**
(21) Application number: 12181650.8
(22) Date of filing: 24.08.2012
(51) Int. Cl.: C07C 29/09, C07C 35/52, C07C 41/16, C07C 43/192, C07C 69/65, C07C 231/02, C07C 233/21, C07C 233/90

(54) **Processes for the preparation of agomelatine and its intermediates**

(30) Priority: 25.08.2011 IN CH29172011
(71) Applicant: Dr. Reddy's Laboratories Ltd., 500 034 Andhra Pradesh (IN)
(72) Inventor: Cherukupally, Praveen, 502313 Andhra Pradesh (IN); Vyala, Sunitha, 500055 Andhra Pradesh (IN); Vujjini, Satish Kumar, Andhra Pradesh (IN); Badarla, Venkata Krishna Rao, 521130 Andhra Pradesh (IN); Kandala, Sreenadha Charyulu, 500072 Andhra Pradesh (IN); Datla, Venkata Rama Krishnam Raju, 500072 Andhra Pradesh (IN); Nalivela, Venu, 502319 Andhra Pradesh (IN)
(74) Representative: Bates, Philip Ian

(57) **Abstract**

Aspects of the present application relate to processes for the preparation of agomelatine and its intermediates which are used in manufacturing process of agomelatine.

## Description

Aspects of the present application relate to processes for the preparation of agomelatine and its intermediates which are used in manufacturing process of agomelatine.

The drug compound having the adopted name "agomelatine" can be represented by structural formula **I.**

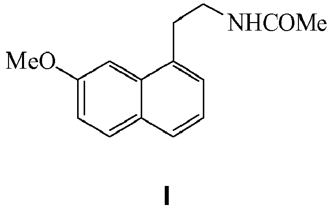

Agomelatine is a non-hygroscopic white or almost white powder, practically insoluble in water and containing no asymmetric carbon atoms. Chemical name for agomelatine is N-[2-(7-methoxy-1-naphthyl)ethyl]acetamide and it is the active ingredient in VALDOXAN^{®} tablets, sold for the treatment of major depressive episodes in adults.

U.S. Patent No. 5,225,442 discloses agomelatine, a pharmaceutically acceptable salt thereof, a pharmaceutical composition thereof, and a method for the treatment of a living animal afflicted with treatable disorder of the melatoninergic system, comprising the step of administering to the living animal an effective amount of the compound. A number of methods for the synthesis of agomelatine have been reported in many patent references such as US 5,225,442, WO 2005/77887 A1, WO 2010/15745 A1, WO 2010/15746 A1, WO 2010/12208 A1, and CN 101973897 A.

However, there remains a need for an environmentally-friendly, cost-effective, and industrially applicable processes for the preparation of agomelatine which alleviates the problems associated with the prior art processes.

### SUMMARY

In an aspect, the application provides a process for the preparation of compound of Formula **V**, which comprises:
a) reacting 2-naphthol with an acetylating agent to provide 7-acetoxynaphthalene, a compound of Formula **II;** 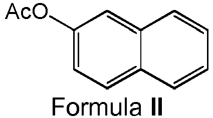
b) reacting the compound of Formula **II** with chloroacetyl chloride to obtain 1-chloroacetyl-7-acetoxynaphthalene, a compound of Formula **III;** 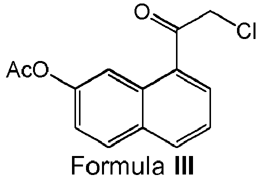
c) reducing the compound of Formula **III** to provide 1-(2-chloroethyl)-7-acetoxynaphthalene, a compound of Formula **IV;** and 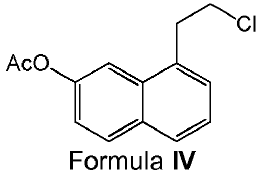
d) hydrolyzing the compound of Formula **IV** followed by methylation with dimethylsulphate to produce the compound of Formula **V**:

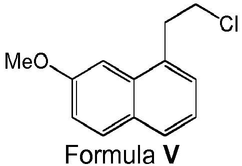

In an aspect, the application provides a process for the preparation of compound of Formula **IVa,** which comprises:
a) reacting 2-naphthol with an acetylating agent to provide 7-acetoxynaphthalene, a compound of Formula **II;** 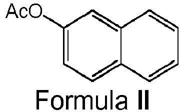
b) reacting the compound of Formula **II** with chloroacetyl chloride to obtain 1-chloroacetyl-7-acetoxynaphthalene, a compound of Formula **III;** 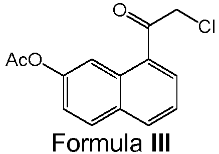
c) reducing the compound of Formula **III** to provide 1-(2-chloroethyl)-7-acetoxynaphthalene, a compound of Formula **IV;** and 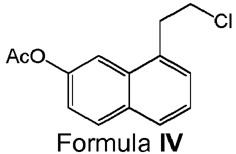
d) deacylating the compound of Formula **IV** to produce 1-(2-chloroethyl)-7-hydroxynaphthalene, a compound of Formula **IVa:**

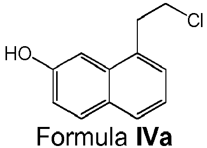

In another aspect, the application provides a process for the preparation of compound of Formula **V** by methylation of compound of Formula **IVa** which is further converted into agomelatine.

In another aspect, the application provides a process for the preparation of 1-(2-aminoethyl)-7-methoxynaphthalene, a compound of Formula **VII** which comprises:
a) reacting the compound of Formula **V** with potassium phthalimide in the presence of a solvent to provide the compound of Formula **VI;** and 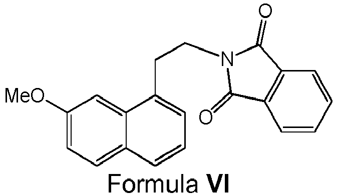
b) hydrolyzing the compound of Formula **VI** to provide 1-(2-aminoethyl)-7-methoxynaphthalene,a compound of Formula **VII:**

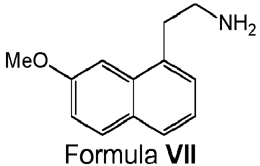

In another aspect, the application provides a process for the preparation of 1-(2-aminoethyl)-7-methoxynaphthalene, a compound of Formula **VII,** which comprises:
a) reacting the compound of Formula **V** with an alkali diformylamide salt to provide a compound of Formula **VIII;** and 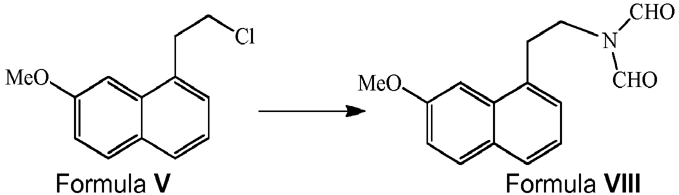
b) hydrolyzing the compound of Formula **VIII** to provide 1-(2-aminoethyl)-7-methoxynaphthalene, a compound of Formula **VII**:

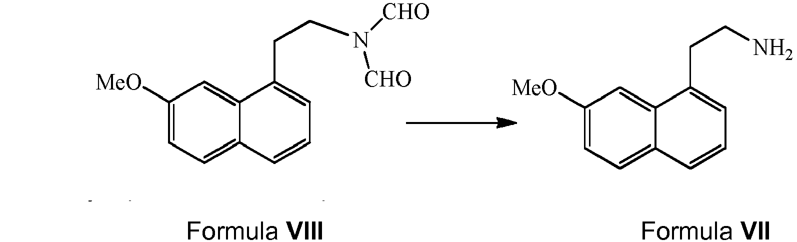

In another aspect, the application provides a process for the preparation of 1-(2-aminoethyl)-7-methoxynaphthalene, a compound of Formula **VII,** which comprises:
a) reacting the compound of Formula **V** with a nitrite salt in the presence of a solvent to provide the compound of Formula **IX;** and 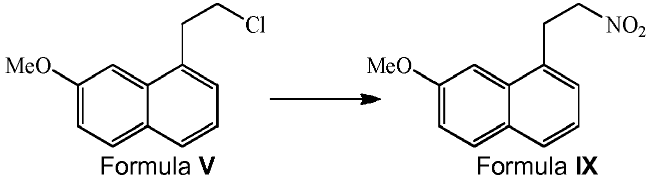
b) reducing the compound of Formula **IX** to provide 1-(2-aminoethyl)-7-methoxynaphthalene of Formula **VII**:

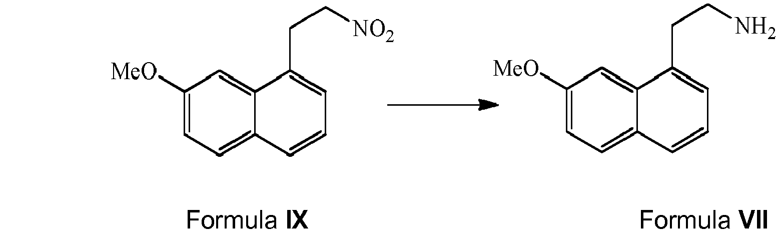

In another aspect, the application provides a process for the preparation of agomelatine, which comprises reacting the 1-(2-aminoethyl)-7-methoxynaphthalene of Formula **VII** prepared by any of the processes describes above, with an acetylating agent to provide agomelatine of Formula **I:**

In another aspect, the application provides a process for the preparation of agomelatine, which comprises:
a) preparing 1-chloroacetyl-7-acetoxynaphthalene from 7-acetoxynaphthalene; and
b) converting 1-chloroacetyl-7-acetoxynaphthalene into aglomelatine.

In another aspect, the application provides a process for the preparation of agomelatine, which comprises:
a) preparing 1-chloroacetyl-7-acetoxynaphthalene from 7-acetoxynaphthalene;
b) reducing 1-chloroacetyl-7-acetoxynaphthalene to produce 1-(2-chloroethyl)-7-acetoxynaphthalene; and
c) converting 1-(2-chloroethyl)-7-acetoxynaphthalene into aglomelatine.

### DETAILED DESCRIPTION

One aspect of the application provides a compound of Formula **III.**

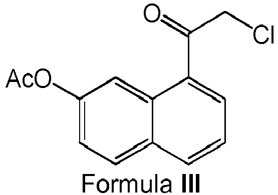

In another aspect, the application provides a compound of Formula **IIIa.** 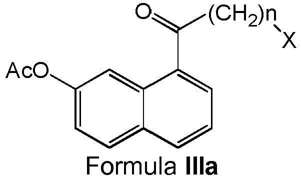 wherein n=1-5 and X = F, Cl, Br, or I

In another aspect, the application provides a compound of Formula **IV.**

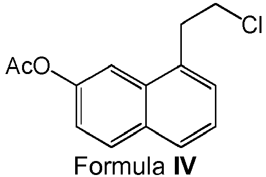

In another aspect, the application provides a compound of Formula **VIII.**

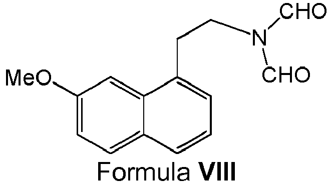

In another aspect, the application provides a compound of Formula **IX.**

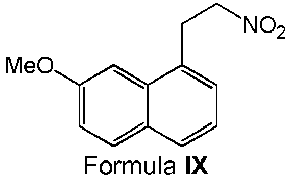

In an aspect, the application provides a process for the preparation of compound of Formula **V**, which comprises:
a) reacting 2-naphthol with an acetylating agent to provide 7-acetoxynaphthalene, a compound of Formula **II;** 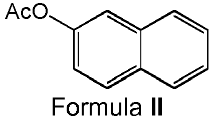
b) reacting the compound of Formula **II** with chloroacetyl chloride to obtain 1-chloroacetyl-7-acetoxynaphthalene, a compound of Formula **III;** 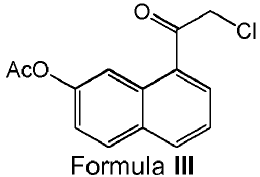
c) reducing the compound of Formula **III** to provide 1-(2-chloroethyl)-7-acetoxynaphthalene, a compound of Formula **IV;** and 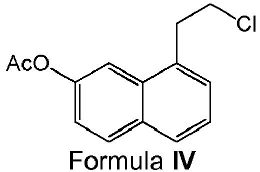
d) hydrolyzing the compound of Formula **IV** followed by methylation with dimethylsulphate to produce the compound of Formula **V**:

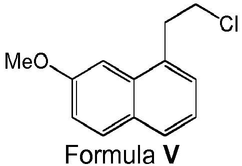

Step a) involves the acetylation of 2-Naphthol to provide 7-acetoxynaphtalene. In embodiments of step a), the acetylation of 2-Naphthol can be carried out in the presence of a base and a solvent. The acetylating agents, which can be used include, but are not limited to, acetic anhydride and acetyl chloride or any acetylating agent which produces compound of Formula **II.**

In embodiments of step a), the acetylation can be carried out in the presence of any suitable inert solvent which are used for any of the acetylation in the literature, preferably acetone, dichloromethane or mixtures thereof.

In embodiments of step a), the acetylation can be carried out in the presence of a base. Bases that are useful in the reaction include, but are not limited to: inorganic bases such as alkali metal or alkaline earth metal carbonates, hydrogen carbonates, hydroxides, carboxylates, *e.g.*, potassium carbonate, potassium hydrogen carbonate, potassium hydroxide, potassium acetate, sodium carbonate, sodium hydrogen carbonate, sodium hydroxide, sodium acetate, or the like; or organic bases such as, for example amines, *e.g.*, triethylamine, N,N-dimethylethanolamine, N,N-diethylethanolamine, 4-ethylmorpholine, 1,4-diazabicyclo[2.2.2]-octane, N-methylmorpholine, dimethylaminopyridine, diisopropylamine, diisopropylethylamine, pyridine, or the like.

In embodiments of step a), the reaction can be carried out at a temperature ranging from about -10°C to about 50°C. In one embodiment, the reaction can be carried out from about -5°C to 40°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step a), the product of step a), may be isolated directly from the reaction mixture itself after the reaction is complete, or after conventional work up with techniques such as quenching with a suitable reagent, extraction, or the like. In embodiments of step a), the product of step-a), i.e., compound of Formula **II** is optionally isolated by extracting in a solvent followed by removal of the solvent by evaporation. In embodiments of step a), optionally, compound of Formula **II** may be used in the next step without isolation.

In embodiments of step a), compound of Formula **II** can be isolated by filtration. The recovered solid i.e., compound of Formula **II** may be optionally further dried at suitable temperatures, and atmospheric or reduced pressures, for about 1-50 hours, or longer, using any types of drying equipment, such as a tray dryer, vacuum oven, air oven, fluidized bed dryer, spin flash dryer, flash dryer or the like.

The obtained compound of Formula **II** may be optionally further purified by recrystallization or by slurrying in a suitable solvent or by column chromatography or any other suitable technique.

Step-b) involves reaction of the compound of Formula **II** with chloroacetyl chloride in the presence of a lewis acid and a solvent to provide the compound of Formula **III.** In embodiments of step b), the reaction of Formula **II** with chloroacetyl chloride can be carried out in the presence of a lewis acid such as anhydrous aluminium chloride (AlCl₃), anhydrous ferric chloride (FeCl₃), boron trifluoride (BF₃), titanium tetrachloride (TiCl₄), zinc chloride (ZnCl₂), stannic chloride (SnCl₄), or the like.

In embodiments of step b), the reaction can be carried out in the presence of a suitable inert solvent which are used for any of the friedel crafts reactions in the literature, preferably acetone, dichloromethane or mixtures thereof.

In embodiments of step b), the reaction can be carried out at a temperature ranging from about -40°C to about 50°C. In one embodiment, the reaction can be carried out from about -30°C to 30°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step b), the product of step b), may be isolated directly from the reaction mixture itself after the reaction is complete, or after conventional work up with techniques such as quenching with a suitable reagent, extraction, or the like. In embodiments of step b), the product of step-b), i.e., compound of Formula **III** is optionally isolated by extracting in a solvent followed by removal of the solvent by evaporation. In embodiments of step b), optionally the compound of Formula **III** may be used in the next step without isolation.

In embodiments of step b), compound of Formula **III** can be isolated by filtration. The recovered solid i.e., compound of Formula **III** may be optionally further dried at suitable temperatures, and atmospheric or reduced pressures, for about 1-50 hours, or longer, using any types of drying equipment, such as a tray dryer, vacuum oven, air oven, fluidized bed dryer, spin flash dryer, flash dryer or the like.

The obtained compound of Formula **III** may be optionally further purified by recrystallization or by slurrying in a suitable solvent or by column chromatography or any other suitable technique.

In embodiments of step b), the product of step-b), i.e., compound of Formula **III** is new and a useful intermediate in the synthesis of agomelatine.

Step-c) involves the reduction of the compound of Formula **III** to provide the compound of Formula **IV.** In embodiments of step-c) the reduction of compound of Formula **III** may be carried out in the presence of a solvent and a reducing agent to provide a compound of Formula **IV.**

In embodiments of step c), the reduction may be carried out by using a suitable reducing agent. The reducing agents which may be used but are not limited to triethylsilane in the presence of titanium tetrachloride, zinc amalgam/concentrated hydrochloric acid, hydrazine in the presence of a base etc.

In embodiments of step c), the reaction may be carried out in a suitable inert solvent. Suitable solvent can be any solvent which has no adverse effect on the reaction or on the reagents involved and that it can dissolve the reagents, at least to some extent. Examples of such solvents include tetrahydrofuran, methanol, dichloromethane, acetonitrile, dimethylformamide, or their mixture thereof.

In embodiments of step c), the reaction can be carried out at a temperature ranging from about -10°C to about boiling point of the solvent. In one embodiment, the reaction can be carried out from about 0°C to room temperature. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step c), the product of step c), may be isolated directly from the reaction mixture itself after the reaction is complete, or after conventional work up with techniques such as quenching with a suitable reagent, extraction, or the like. In embodiments of step c), the product of step-c), i.e., compound of Formula **IV** is optionally isolated by extracting in a solvent followed by removal of the solvent by evaporation. In embodiments of step c), optionally the product of step-c), i.e., compound of Formula **IV** may be used in the next step without isolation.

In embodiments of step c), the product of step-c), i.e., compound of Formula **IV** is new and a useful intermediate in the synthesis of agomelatine.

Step-d) involves the preparation of the compound of Formula **V** by hydrolyzing / deacylating the compound of Formula **IV** followed by methylation.

In embodiments of step d), compound of formula IV is treated with a base and a solvent to produce deacylated compound. In embodiments of step d), the product i.e., deacylated compound of formula **IV** can be treated with methylating agent to produce a compound of formula V.

The bases which can be used in this step include but are not limited to inorganic bases such as alkali metal or alkaline earth metal carbonates, hydrogen carbonates, hydroxides, carboxylates, or alkoxides, *e.g.*, potassium carbonate, potassium hydrogen carbonate, potassium hydroxide, potassium acetate, potassium methoxide, sodium carbonate, sodium hydrogen carbonate, sodium hydroxide, sodium acetate, sodium methoxide, or the like.

In embodiments of step d), the methylation step can be carried by using dimethyl sulphate in the presence of a base and a solvent. The base that is used for the production of deacylated compound also can be useful for this step also. The solvents such as alcohols such as methanol isopropyl alchol; ketones such as acetone, methyl isobutyl ketone; esters such as ethyl acetate; nitriles such as acetonitrile etc. may be used in the reaction. Any of the bases listed in embodiment of step-a) may be used in the reaction.

In embodiments of step d), the reaction can be carried out at a temperature ranging from about 0°C to about boiling point of the solvent. In one embodiment, the reaction can be carried out from about 10°C to 70°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step d), the product of step d), may be isolated directly from the reaction mixture itself after the reaction is complete, or after conventional work up with techniques such as quenching with a suitable reagent, extraction, or the like. In embodiments of step d), the product of step-d), i.e., compound of Formula **V** is optionally isolated by extracting in a solvent followed by removal of the solvent by evaporation. In embodiments of step d), optionally the product of step-d), i.e., compound of Formula **V** may be used in the next step without isolation.

In embodiments of step d), compound of Formula **V** can be isolated by filtration. The recovered solid i.e., compound of Formula **V** may be optionally further dried at suitable temperatures, and atmospheric or reduced pressures, for about 1-50 hours, or longer, using any types of drying equipment, such as a tray dryer, vacuum oven, air oven, fluidized bed dryer, spin flash dryer, flash dryer or the like.

The obtained compound of Formula **V** may be optionally further purified by recrystallization or by slurrying in a suitable solvent or by column chromatography or any other suitable technique.

In another aspect, the application provides process for the preparation of compound of Formula **IIIA.** In embodiments, the compound of Formula **IIIA** can be prepared by the same procedure as described for the preparation of compound of Formula **III** herein using appropriate acid chloride.

In an aspect, the application provides a process for the preparation of compound of Formula **IVa,** which comprises:
a) reacting 2-naphthol with an acetylating agent to provide 7-acetoxynaphthalene, a compound of Formula **II;** 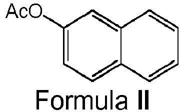
b) reacting the compound of Formula **II** with chloroacetyl chloride to obtain 1-chloroacetyl-7-acetoxynaphthalene, a compound of Formula **III**; 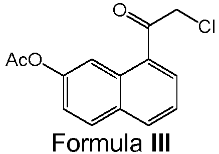
c) reducing the compound of Formula **III** to provide 1-(2-chloroethyl)-7-acetoxynaphthalene, a compound of Formula **IV;** and 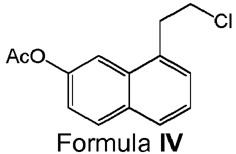
d) deacylating the compound of Formula **IV** to produce 1-(2-chloroethyl)-7-hydroxynaphthalene, a compound of Formula **IVa;**

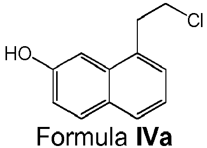

In embodiments, step a), step b) and step c) can be performed according to the procedures as described above in the present application.

Step d) involves the hydrolysis / deacylation of the compound of Formula **IV** to provide the compound of formula **IVa.** In embodiments of step d) the deacylation can be carried out in the presence of a base and a solvent. The solvents such as alcohols such as methanol isopropyl alcohol; ketones such as acetone, methyl isobutyl ketone; esters such as ethyl acetate; nitriles such as acetonitrile etc. may be used in the reaction. Any of the bases listed in embodiment of step-a) may be used in the reaction.

In embodiments of step d), the product of step-d), i.e., compound of Formula **IVa** is optionally isolated by extracting in a solvent followed by removal of the solvent by evaporation. In embodiments of step d), optionally the product of step-d), i.e., compound of Formula **IVa** may be used in the next step without isolation.

In embodiments of step d), compound of Formula **IVa** can be isolated by filtration. The recovered solid i.e., compound of Formula **IVa** may be optionally further dried at suitable temperatures, and atmospheric or reduced pressures, for about 1-50 hours, or longer, using any types of drying equipment, such as a tray dryer, vacuum oven, air oven, fluidized bed dryer, spin flash dryer, flash dryer or the like.

The obtained compound of Formula **IVa** may be optionally further purified by recrystallization or by slurrying in a suitable solvent or by column chromatography or any other suitable technique.

In another aspect, the application provides a process for the preparation of compound of Formula **V** by methylation of compound of Formula **IVa.** In another aspect, the application provides a process for the preparation of agomelatine by converting the compound of Formula **IVa** to compound of Formula **V** which is further converted to Agomelatine by the procedure described in the present application.

In an embodiment, it involves the preparation the compound of compound of Formula **V** by methylation of compound of Formula **IVa** with a methylating agent. In embodiments, the methylation can be carried out with dimethylsulfate. In embodiments, the methylation can be carried out in the presence of a base and a solvent. Any of the base and solvent listed in step d) of the above aspect can be used in this step. The compound of Formula **V** can be purified and dried according to the procedure as described in the present application.

In another aspect, the application provides process for the preparation of compound of Formula **Va.** 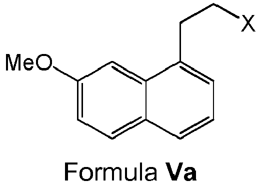 Wherein X = F, Cl, Br or I

In embodiments, the compound of Formula **Va** can be prepared by the same procedure as described for the preparation of compound of Formula **V** herein using appropriate methods.

In another aspect, the application provides a process for the preparation of 1-(2-aminoethyl)-7-methoxynaphthalene compound of Formula **VII** which comprises:
a) reacting the compound of Formula **V** with potassium phthalimide in the presence of a solvent to provide the compound of Formula **VI;** and 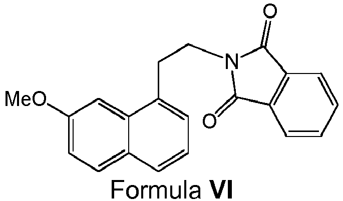
b) hydrolyzing the compound of Formula **VI** to provide 1-(2-aminoethyl)-7-methoxynaphthalene compound of Formula **VII:**

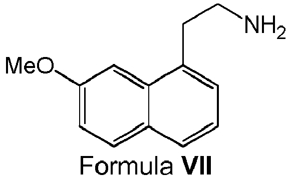

In embodiments of step a), the reaction of compound of Formula **V** can be carried out with potassium phthalimide in the presence of a solvent to provide the compound of Formula **VI.** Examples of such solvents include, but are not limited to: ethers, such as diethyl ether, diisopropyl ether, tetrahydrofuran, dioxane and dimethoxyethane; ketones, such as acetone, methyl ethyl ketone, methyl isobutyl ketone, and diethyl ketone; esters, such as ethyl acetate, propyl acetate, isopropyl acetate, and butyl acetate; alcohols, such as methanol, ethanol, ethylene glycol, 1-propanol, 2-propanol, 2-methoxyethanol, 1-butanol, 2-butanol, iso-butyl alcohol, t-butyl alcohol, 2-ethoxyethanol, diethylene glycol, 1-, 2-, or 3-pentanol, cyclohexanol, benzyl alcohol, phenol, glycerol, and C₁-C₆ alcohols; nitriles, such as acetonitrile and propionitrile; amides, such as formamide, N,N-dimethylformamide, N,N-dimethylacetamide, N-methyl-2-pyrrolidone, and hexamethyl phosphoric triamide; sulfoxides, such as dimethylsulfoxide; halogenated hydrocarbons, such as dichloromethane, chloroform and chlorobenzene; aromatic hydrocarbons, such as toluene; any mixtures of two or more thereof.

In embodiments of step a), the reaction can be carried out at a temperature ranging from about 10°C to about boiling temperature of the solvent. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step a), the product of step a), may be isolated directly from the reaction mixture itself after the reaction is complete, or after conventional work up with techniques such as quenching with a suitable reagent, extraction, or the like. In embodiments of step a), the product of step-a), i.e., compound of Formula **VI** is isolated by filtration of the precipitated product.

The recovered solid i.e., compound of Formula **VI** may be optionally further dried at suitable temperatures, and atmospheric or reduced pressures, for about 1-50 hours, or longer, using any types of drying equipment, such as a tray dryer, vacuum oven, air oven, fluidized bed dryer, spin flash dryer, flash dryer or the like.

The obtained compound of Formula **VI** may be optionally further purified by recrystallization or by slurrying in a suitable solvent or by column chromatography or any other suitable technique.

Step-b) involves the hydrolysis of the compound of Formula **VI** to provide the compound of Formula **VII.**

In embodiments of step b), the hydrolysis of compound of Formula **VI** can be carried out in the presence of acid or base to provide the compound of Formula **VII**.

In embodiments of step b), the bases which can be used for the hydrolysis is selected from alkali metal hydroxide such as sodium hydroxide, potassium hydroxide; alkaline-earth metal hydroxide such as magnesium hydroxide, calcium hydroxide, or hydrazine hydrate. In embodiments of step b), the hydrolysis can be carried out in the presence of acid such as hydrochloric acid, sulfuric acid etc.

In embodiments of step b), the reaction can be carried out at a temperature ranging from about 10°C to about boiling temperature of the solvent. In one embodiment, the reaction can be carried out from about 20°C to 90°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step b), the product of step b), may be isolated directly from the reaction mixture itself after the reaction is complete, or after conventional work up with techniques such as quenching with a suitable reagent, extraction, or the like. In embodiments of step b), the product of step-b), i.e., compound of Formula **VII** is optionally isolated by extracting in a solvent followed by removal of the solvent by evaporation.

In embodiments of step b), the product of step-b), i.e., compound of Formula **VII** obtained from the above process can be used for the preparation of agomelatine.

In another aspect, the application provides a process for the preparation of 1-(2-aminoethyl)-7-methoxynaphthalene compound of Formula **VII,** which comprises:
a) reacting the compound of Formula **V** with an alkali diformylamide salt to provide a compound of Formula **VIII;** and 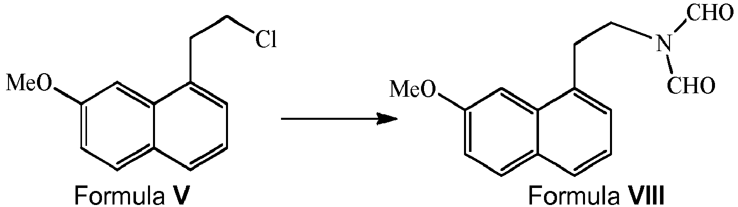
b) hydrolyzing the compound of Formula **VIII** to provide 1-(2-aminoethyl)-7-methoxynaphthalene of Formula **VII:**

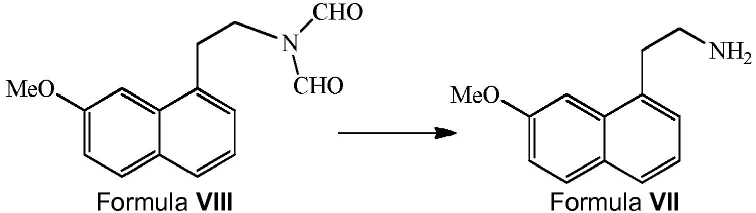

Step-a) involves the preparation of compound of Formula **VIII** by the reaction of compound of Formula **V** with sodium diformyl amide. In embodiments of step a), sodium diformyl amide used in the reaction can be prepared by reacting formamide with sodium methoxide.

In embodiments of step a), the reaction can be carried out in the presence or absence of a suitable solvent. Examples of such solvents include, but are not limited to: ethers, such as diethyl ether, diisopropyl ether, tetrahydrofuran, dioxane, and dimethoxyethane; ketones, such as acetone, methyl ethyl ketone, methyl isobutyl ketone, and diethyl ketone; esters, such as ethyl acetate, propyl acetate, isopropyl acetate, and butyl acetate; alcohols, such as methanol, ethanol, ethylene glycol, 1-propanol, 2-propanol, 2-methoxyethanol, 1-butanol, 2-butanol, iso-butyl alcohol, t-butyl alcohol, 2-ethoxyethanol, diethylene glycol, 1-, 2-, or 3-pentanol, cyclohexanol, benzyl alcohol, phenol, glycerol, and C₁-C₆ alcohols; nitriles, such as acetonitrile and propionitrile; amides, such as formamide, N,N-dimethylformamide, N,N-dimethylacetamide, N-methyl-2-pyrrolidone, and hexamethyl phosphoric triamide; sulfoxides such as dimethylsulfoxide; halogenated hydrocarbons, such as dichloromethane, chloroform, carbon tetrachloride, and chlorobenzene; aromatic hydrocarbons, such as toluene; any mixtures of two or more thereof.

In embodiments of step a), the reaction can be carried out at a temperature ranging from about 10°C to about boiling temperature of the solvent. In one embodiment, the reaction can be carried out from about 20°C to 110°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step a), the product of step a), may be isolated directly from the reaction mixture itself after the reaction is complete, or after conventional work up with techniques such as quenching with a suitable reagent, extraction, or the like. In embodiments of step a), the product of step-a), i.e., compound of Formula **VIII** is optionally isolated by extracting in a solvent followed by removal of the solvent by evaporation.

In embodiments of step a), the product of step-a), i.e., compound of Formula **VIII** is new and useful intermediate in the synthesis of agomelatine.

In embodiments of step a) the product obtained is a mixture of monoformylated product (N-[2-(7-methoxy-1-naphthyl)ethyl]formamide and compound of Formula **VIII**. In embodiments, the product which is a mixture of monoformylated product and compound of Formula **VIII** may be formed in any ratio based on the reaction condition however both these intermediates are converted into compound of Formula **VII**.

Step b) involves the hydrolysis the compound of Formula **VIII** to provide 1-(2-aminoethyl)-7-methoxynaphthalene.

In embodiments of step b), the hydrolysis can be carried out in the presence of acid in a solvent. The acid can be used in the reaction selected from hydrochloric acid, sulfuric acid or the like. Any of the solvent listed in step-a) can be used in the reaction. In embodiments of step b), the hydrolysis can also be carried out in the presence of base in a solvent. Any of the base and solvent described herein can be used in the reaction.

In embodiments of step b), the reaction can be carried out at a temperature ranging from about 10°C to about boiling temperature of the solvent. In one embodiment, the reaction can be carried out at a temperature from about 20°C to 90°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step b), the product of step b), may be isolated directly from the reaction mixture itself after the reaction is complete, or after conventional work up with techniques such as quenching with a suitable reagent, extraction, or the like. In embodiments of step b), the product of step-b), i.e., compound of Formula **VII** is optionally isolated by extracting in a solvent followed by removal of the solvent by evaporation. The compound of Formula **VII** can be isolated or can be converted to Agomelatine of Formula **I** without isolation.

In another aspect, the application provides a process for the preparation of 1-(2-aminoethyl)-7-methoxynaphthalene, a compound of Formula **VII,** which comprises:
a) reacting the compound of Formula **V** with a nitrite salt in the presence of a solvent to provide the compound of Formula **IX;** and 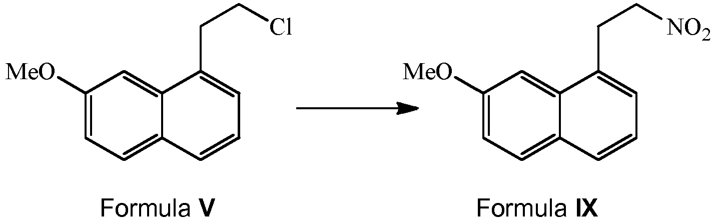
b) reducing the compound of Formula **IX** to provide 1-(2-aminoethyl)-7-methoxynaphthalene of Formula **VII:**

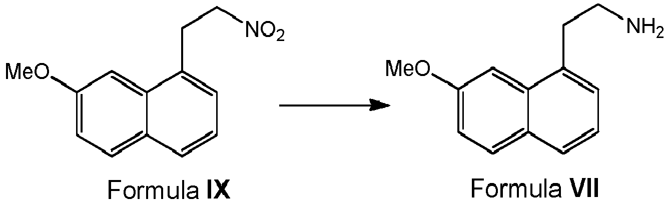

Step-a) involves the preparation of compound of Formula **IX** by the reaction of compound of Formula **V** with a nitrite salt in the presence of a solvent. Suitable agents for displacement of halogen by nitrite used in step a) include, but are not limited to metallic nitrites, such as sodium nitrite, potassium nitrite, lithium nitrite, silver nitrite, or like. In embodiments of step a), the compound of Formula **IX** can be prepared by the reaction of compound of Formula **V** sodium nitrite. In embodiments of step a), the reaction can be carried out in the presence of a polar solvent. In embodiments of step a), the reaction can be carried out in the presence of solvents such as dimethylformamide, dimethylacetamide, dimethylsulfoxide, acetonitrile or the like.

In embodiments of step a), the reaction can be carried out at a temperature ranging from about 10°C to about boiling temperature of the solvent. In one embodiment, the reaction can be carried out from about 20°C to 110°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step a), the product of step a), may be isolated directly from the reaction mixture itself after the reaction is complete, or after conventional work up with techniques such as quenching with a suitable reagent, extraction, or the like. In embodiments of step a), the product of step-a), i.e., compound of Formula **IX** is optionally isolated by extracting in a solvent followed by removal of the solvent by evaporation.

In embodiments of step a), the product of step-a), i.e., compound of Formula **IX** is new and useful intermediate in the synthesis of agomelatine.

Step-b) involves the preparation of compound of Formula **VII** by the reduction of compound of Formula **IX**. In embodiments of step b), the reduction may be carried out in the presence of a suitable reducing agent and a solvent. In embodiments of step b), the reducing agents that may be used but are not limited to palladium on carbon, raney nickel or the like.

In embodiments of step b), the reaction can be carried out in the presence of a suitable solvent. Examples of such solvents include, but are not limited to: ethers, such as diethyl ether, diisopropyl ether, tetrahydrofuran, dioxane, and dimethoxyethane; alcohols, such as methanol, ethanol, ethylene glycol, 1-propanol, 2-propanol, 2-methoxyethanol, 1-butanol, 2-butanol, iso-butyl alcohol, t-butyl alcohol, glycerol, and C₁-C₆ alcohols; halogenated hydrocarbons, such as dichloromethane, chloroform, carbon tetrachloride, and chlorobenzene; aromatic hydrocarbons, such as toluene; any mixtures of two or more thereof.

In embodiments of step b), the reaction can be carried out at a temperature ranging from about 0°C to about boiling temperature of the solvent. In one embodiment, the reaction can be carried out from about 10°C to 70°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of step b), the product of step b), may be isolated directly from the reaction mixture itself after the reaction is complete, or after conventional work up with techniques such as quenching with a suitable reagent, extraction, or the like. In embodiments of step b), the product of step-b), i.e., compound of Formula **VII** is optionally isolated by extracting in a solvent followed by removal of the solvent by evaporation. The compound of Formula **VII** can be isolated or can be converted to agomelatine of Formula **I** without isolation.

In an embodiment, 1-(2-aminoethyl)-7-methoxynaphthalene obtained in any of the processes described above can be used as such or can be isolated directly from the reaction as a pharmaceutically acceptable salt or can be converted into a pharmaceutically acceptable salt. The pharmaceutically acceptable salts of 1-(2-aminoethyl)-7-methoxynaphthalene can be converted 1-(2-aminoethyl)-7-methoxy naphthalene by treatment with a base whenever required or before or during the next step. The bases that are described in the present application can be used for this conversion step. The acids which can be used for the salt preparation are mineral acids such as hydrochloric acid, hydrobromic acid, sulfuric acid etc.

In another aspect, the application provides a process for the preparation of agomelatine, which further comprises reacting the 1-(2-aminoethyl)-7-methoxynaphthalene of Formula **VII** prepared by any of the processes describes above with an acetylating agent to provide agomelatine of Formula I.

The process involves the preparation of agomelatine by acetylating 1-(2-aminoethyl)-7-methoxynaphthalene compound of Formula **VII** with a suitable acetylating agent. The acetylating agents, which can be used include, but are not limited to, acetic anhydride and acetyl chloride or any acetylating agent which produces agomelatine. In embodiments, the acetylation can be carried out in the presence of a base and a solvent. Examples of such solvents include, but are not limited to: ethers, such as diethyl ether, diisopropyl ether, tetrahydrofuran, dioxane, and dimethoxyethane; ketones, such as acetone, methyl ethyl ketone, methyl isobutyl ketone, and diethyl ketone; esters, such as ethyl acetate, propyl acetate, isopropyl acetate; alcohols, such as methanol, ethanol, ethylene glycol, 1-propanol, 2-propanol, 2-methoxyethanol, 1-butanol, 2-butanol, iso-butyl alcohol, t-butyl alcohol, 2-ethoxyethanol, diethylene glycol, cyclohexanol, benzyl alcohol, phenol, glycerol, and C₁-C₆ alcohols; nitriles, such as acetonitrile and propionitrile; amides, such as formamide, N,N-dimethylformamide, N,N-dimethylacetamide and N-methyl-2-pyrrolidone; sulfoxides, such as dimethylsulfoxide; halogenated hydrocarbons, such as dichloromethane, chloroform, carbon tetrachloride, and chlorobenzene; aromatic hydrocarbons, such as toluene; any mixtures of two or more thereof.

In embodiments of the process, the acetylation can be carried out in the presence of a base. Bases that are useful in the reaction include, but are not limited to: inorganic bases such as alkali metal or alkaline earth metal carbonates, hydrogen carbonates, hydroxides, carboxylates, or alkoxides, *e.g.*, potassium carbonate, potassium hydrogen carbonate, potassium hydroxide, potassium acetate, potassium methoxide, sodium carbonate, sodium hydrogen carbonate, sodium hydroxide, sodium acetate, sodium methoxide, barium hydroxide, calcium oxide, or alkali metal hydrides *e.g.* sodium hydride, potassium hydride, or the like; or organic bases such as, for example amines, *e.g.*, triethylamine, N,N-dimethylethanolamine, N,N-diethylethanolamine, 4-ethylmorpholine, N-methylmorpholine, dimethylaminopyridine, diisopropylamine, diisopropylethylamine, pyridine, or the like.

In embodiments of the process, the reaction can be carried out at a temperature ranging from about -10°C to about boiling point of the solvent. In one embodiment, the reaction can be carried out from about 10°C to 80°C. The time required for the reaction may also vary widely, depending on many factors, notably the reaction temperature and the nature of the reagents and solvent employed. However, provided that the reaction is effected under the conditions outlined above, for a period of about 1 to about 24 hours or longer.

In embodiments of the process, the obtained agomelatine may be isolated directly from the reaction mixture itself after the reaction is complete, or after conventional work up with techniques such as quenching with a suitable reagent, extraction, or the like. In embodiments of the process, the obtained agomelatine may be recrystallized to obtain pure agomelatine. In embodiments, the agomelatine as obtained may be optionally further dried at suitable temperatures, and atmospheric or reduced pressures, for about 1-50 hours, or longer, using any types of drying equipment, such as a tray dryer, vacuum oven, air oven, fluidized bed dryer, spin flash dryer, flash dryer or the like.

In another aspect, the application provides a process for the preparation of agomelatine, which comprises:
a) preparing 1-chloroacetyl-7-acetoxynaphthalene from 7-acetoxynaphthalene; and
b) converting 1-chloroacetyl-7-acetoxynaphthalene into aglomelatine.

In another aspect, the application provides a process for the preparation of agomelatine, which comprises:
a) preparing 1-chloroacetyl-7-acetoxynaphthalene from 7-acetoxynaphthalene;
b) reducing 1-chloroacetyl-7-acetoxynaphthalene to produce 1-(2-chloroethyl)-7-acetoxynaphthalene; and
c) converting 1-(2-chloroethyl)-7-acetoxynaphthalene into aglomelatine.

The potential impurities possible in agomelatine are the unreacted starting materials and intermediates as described in the present application. The possible impurities in agomelatine in addition to the unreacted starting materials or intermediates described in the present application can have structural formulas as illustrated below,

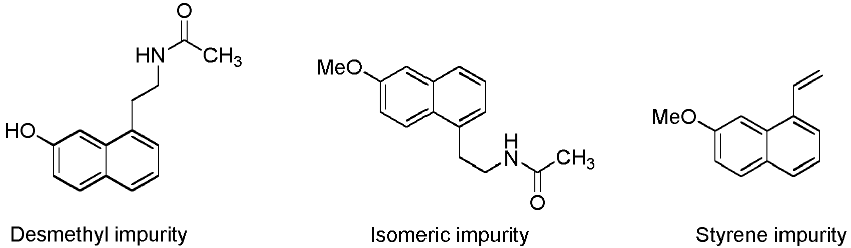

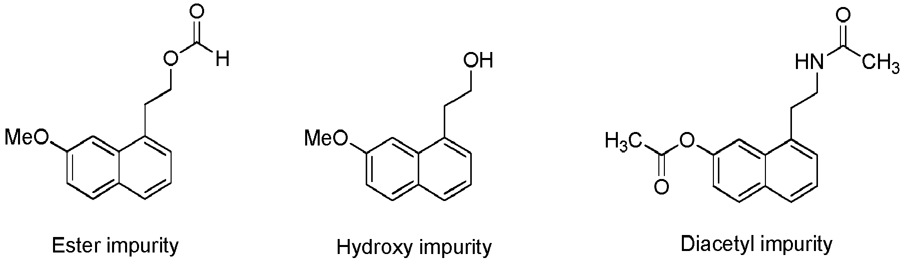

In another aspect, the application relates to substantially pure agomelatine having less than about 0.1% of each of the Desmethyl impurity, Isomeric impurity, Styrene impurity, Ester impurity, Hydroxy impurity and Diacetyl impurity. In another aspect, the application provides processes for preparing agomelatine having less than about 0.1% of the Desmethyl impurity, Isomeric impurity, Styrene impurity, Ester impurity, Hydroxy impurity and Diacetyl impurity.

In embodiments, prepared agomelatine according to the processes of the present application can be substantially pure having a chemical purity greater than about 99%, or greater than about 99.5%, or greater than about 99.9%, by weight, as determined using high performance liquid chromatography (HPLC). Agomelatine produced by the methods of present application can be chemically pure agomelatine having purity greater than about 99.5% and containing no single impurity in amounts greater than about 0.15%, by HPLC. Agomelatine produced by methods of present application can be chemically pure agomelatine having purity greater than about 99.8% and containing no single impurity in amounts greater than about 0.1%, by HPLC.

In embodiments, agomelatine or any of the intermediates described herein obtained from any of the processes described herein can be purified by any method known in the art such as recrystallization involving single solvent, mixture of solvents or solvent-anti-solvent technique; slurring in a solvent; or chromatography to improve its chemical purity.

Agomelatine obtained according to the processes of the present application can be milled or micronized by any process known in the art, such as ball milling, jet milling, wet milling etc., to produce desired particle sizes and particle size distributions. The bulk density, particle size and hausner ratio of agomelatine produced according to the processes of the present application will vary depending upon the conditions used. These properties of agomelatine will vary from the product directly produced according to the processes disclosed in this application and after milling / micronization step.

The agomelatine obtained according to any of the processes of the present application may be in crystalline or amorphous or solvate or hydrate or mixture of the crystalline forms. The starting materials of the present application can be produced according to any of the processes known in the literature. The starting materials may be purified according to the known methods before using for the reaction. The agomelatine obtained according to the any of the processes described in the present application can be used in the pharmaceutical composition together with pharmaceutically acceptable excipients.

Certain specific aspects and embodiments of the present application will be explained in greater detail with reference to the following examples, which are provided only for purposes of illustration and should not be construed as limiting the scope of the application in any manner. Variations of the described procedures, as will be apparent to those skilled in the art, are intended to be within the scope of the present application.

### EXAMPLES

**Example 1: Preparation of 7-acetoxynaphtalene (compound of Formula II):** 2-naphthol (5 g), triethylamine (4.8 mL), and dichloromethane (25 mL) are charged into a round bottom flask at 28°C. The mixture is stirred for 10 minutes at 28°C to obtain a clear solution. The reaction mass is cooled to 5°C. Dimethylaminopyridine (0.01 g) is added to the reaction mass. Acetic anhydride (3.2 mL) is added dropwise to the reaction mass at 5°C in 10 minutes. The reaction mass is stirred for 40 minutes at 5°C. The temperature of the reaction mass is raised to 25°C and stirred for 45 minutes at the same temperature. Again, acetic anhydride (0.5 equivalents) is added to the reaction mass to complete the reaction. Water (50 mL) is added to the reaction mass and stirred for 15 minutes at 25°C. The layers are separated and organic layer is washed with water (50 mL). The organic layer is evaporated under vacuum to get the title compound as residue.

**Example 2: Preparation of 1-chloroacetyl-7-acetoxynaphthalene (compound of Formula III):** 7-acetoxynaphthalene (25 g) and dichloromethane (500 mL) are charged into a round bottom flask at 26°C and stirred for 15 minutes at the same temperature. The reaction mass is cooled to 5°C. Aluminium chloride (80.4 g) is charged to the reaction mass at 5°C. The reaction mass is further cooled to -20°C. Chloroacetyl chloride (16 mL) is added dropwise to the reaction mass in 1 hour at - 20°C. The reaction mass is maintained for 4 hours at -20°C. A 2 N hydrochloric acid solution is added dropwise to the reaction mass in 30 minutes at -20°C. Dichloromethane (200 mL) is added to the reaction mass and stirred for 20 minutes. The layers are separated and the organic layer is washed with saturated solution of sodium bicarbonate (400 mL). The organic layer is evaporated completely to obtain a residue. Ethyl acetate (25 mL) is added to the residue and stirred to get clear solution. The obtained solution is cooled to 5°C, hexane (500 mL) is added, and stirred for 2 hours at 5°C. The solvent is decanted, ethyl acetate (25 mL) is added to the residue, and stirred at 40°C until the residue dissolves. The obtained solution is cooled to 5°C. Hexane (500 mL) is added and stirred for 4 hours at 5°C. The precipitated solid compound is obtained by filtration and washed with hexane (30 mL). The material is dried under suction. Yield: 17 g.

**Example 3: Preparation of 1-(2-chloroethyl)-7-acetoxynaphthalene (compound of Formula IV):** 1-chloroacetyl-7-acetoxynaphthalene (20 g) and dichloromethane (100 mL) are charged into a round bottom flask at 30°C. Triethylsilane (29.1 mL) is added to the reaction mass dropwise in 10 minutes at 30°C. The reaction mass is cooled to 0°C and titanium tetrachloride (17 mL) diluted with dichloromethane (10 mL) is added dropwise at 5°C. The reaction mass is maintained for 2 hours at 30°C. The reaction mass is cooled to 0°C and dichloromethane (100 mL) is added to the reaction mass. Saturated solution of sodium bicarbonate (100 mL) is added dropwise to the reaction mass slowly in 1 hour at 5°C. The reaction mass is filtered. The layers are separated and the aqueous layer is extracted with dichloromethane (100 mL). The combined organic layer is washed with saturated solution of sodium bicarbonate (100 mL). The organic layer is dried on sodium sulphate and evaporated on a Rotavapour™ to obtain a residue. The residue is dissolved in acetonitrile (100 mL) and hexane (100 mL) is added to the solution at 30°C. The layers are separated and aectonitrile layer is washed with hexane (100 mL). The acetonitrile layer is evaporated to obtain the title compound as residue. Yield: 18 g.

**Example 4: Preparation of 1-(2-chloroethyl)-7-methoxynaphthalene (compound of Formula V):** 1-(2-chloroethyl)-7-acetoxynaphthalene (18 g), potassium carbonate (30 g), and methanol (144 ml) are charged into a round bottom flask at 30°C. Dimethyl sulphate (25.9 mL) is added slowly in 10 minutes at 30°C. The reaction mass is heated to 60°C and maintained for 1 hour 30 minutes at the same temperature. The reaction mass is cooled to 30°C. Water (90 mL) and dichloromethane (90 mL) are added to the reaction mass at 30°C and stirred for 10 minutes. The layers are separated and the aqueous layer is extracted with dichloromethane (90 mL). The combined organic layer is washed with water (90 mL), with brine solution (100 mL), and dried over sodium sulphate. The organic layer is evaporated to a residue. The residue is purified by column chromatography. Yield: 9.2 g.

**Example 5: Preparation of 2-(2-(7-methoxynaphthalen-1-yl)ethyl) isoindoline-1,3-dione (compound of Formula VI):** 1-(2-chloroethyl)-7-methoxynaphthalene (9.2 g) and dimethylformamide (92 mL) are charged into a round bottom flask at 30°C. Potassium phthalimide (15.4 g) and potassium iodide (0.2 g) are added to the reaction mass at 30°C. The reaction mass is heated to 102°C and maintained for 2 hours at the same temperature. The reaction mass is cooled to 30°C, water (360 mL) is added to the reaction mass, and stirred for 30 minutes at 30°C. The precipitated solid is collected by filtration. The solid compound is slurried in a mixture of ethyl acetate (8 mL) and hexane (400 mL), filtered, and washed with hexane. The compound is dried under suction. Yield: 13 g.

**Example 6: Preparation of 1-(2-aminoethyl)-7-methoxynaphthalene (compound of Formula VII):** 2-(2-(7-methoxynaphthalen-1-yl)ethyl)isoindoline-1,3-dione (15 g) and methanol (150 mL) are charged into a round bottom flask at 30°C. The reaction mass is stirred for 10 minutes at 30°C and hydrazine hydrate (19.4 mL) is added to the reaction mass at the same temperature. The reaction mass is heated to 65°C and maintained for 1 hour at the same temperature. The reaction mass is cooled to 30°C. The reaction mass is filtered and washed with dichloromethane (100 mL). Water (100 mL) is added to the filtrate and extracted with dichloromethane (3 X 100 mL). The layers are separated; the organic layer is washed with brine, and dried over sodium sulphate. The organic layer is evaporated to obtain the title compound as oil.

**Example 7: Preparation of 1-(N, N-diformylaminoethyl)-7- methoxynaphthalene (compound of Formula VIII):** formamide (10.2 g) and sodium methoxide (6 g) are charged into a round bottom flask at 30°C. The reaction mass is stirred for 3 hours at 30°C and then heated to 80°C and stirred for 1 hour 30 minutes. 1-(2-Chloroethyl)-7-methoxynaphthalene (5 g) dissolved in dimethyl formamide (50 mL) and catalytic amount of tetrabutylammonium iodide are added to the reaction mass at 80°C. The reaction mass is stirred for 30 minutes at 80°C. The reaction mass is heated to 100°C and stirred for 4 hours. The reaction mass is cooled to 30°C after completion of the reaction and water (100 mL) is added. Ethyl acetate (150 mL) is added to the reaction mass and stirred for 5 minutes at 30°C. The layers are separated and the aqueous layer is extracted with ethyl acetate (100 mL). The combined organic layer is washed with brine solution and dried over sodium sulphate. The solvent from the organic layer is evaporated to obtain the compound as crude. Yield: 6.7 g (wet).

**Example 8: Preparation of 1-(2-aminoethyl)-7-methoxynaphthalene (compound of Formula VII):** 1-(N, N-diformylaminoethyl)-7-methoxynaphthalene (6.1 g) and methanol (60 mL) are charged into a round bottom flask at 30°C. Concentrate hydrochloric acid (12 mL, 36.5%) is added to the reaction mass at 30°C. The reaction mass is heated to 70°C and stirred for 4 hours at the same temperature. The reaction mass is cooled to 30°C after completion of the reaction and concentrated under vacuum to remove the solvent. The obtained solid is stirred in a mixture of ethyl acetate and hexane (40+60 mL) for 3 hours at 30°C. The title compound as a hydrochloride salt is obtained by filtration and washed with hexane. Yield: 5 g.

**Example 9: Preparation of 1-(2-nitroethyl)-7-methoxynaphthalene (compound of Formula IX):** 1-(2-chloroethyl)-7-methoxynaphthalene (1 g) and dimethylformamide (10 mL) are charged into a round bottom flask at 30°C. Sodium nitrite (0.62 g), phloroglucinol (0.17 g), and tetrabutylammonium iodide (0.2 g) are added to the reaction mass at 30°C.The reaction mass is heated to 90°C and stirred for 3 hours at the same temperature. The reaction mass is cooled to 30°C after completion of the reaction and water (20 mL) is added. The reaction mass is extracted with ethyl acetate (2 X 20 mL). The combine organic layer is washed with water (20 mL) and brine (20 mL). The organic layer is dried over sodium sulphate and evaporated the solvent to obtain the product as a residue. Yield: 1 g.

**Example 10: Preparation of 1-(2-aminoethyl)-7-methoxynaphthalene (compound of Formula VII):** 1-(2-nitroethyl)-7-methoxynaphthalene (1 g) and methanol (40 mL) are charged into an autoclave at 30°C. Raney™ nickel (1 g) is added to the reaction mass at 30°C. The reaction mass is heated to 50°C and stirred for 5 hours under hydrogen pressure at 50°C. The reaction mass is cooled to 30°C after completion of the reaction. The reaction mass is filtered and washed with methanol (100 mL). The filtrate is concentrated under vacuum to obtain the title compound as residue. Yield: 0.9 g.

**Example 11: Preparation of N-[2-(7-methoxy-1-naphthylen-1-yl)ethyl]acetamide (agomelatine):** 1-(2-aminoethyl)-7-methoxynaphthalene (5.1 g) and ethanol (40 mL) are charged into a round bottom flask at 30°C. Sodium acetate (2.51 g) is added to the reaction mass at 30°C. Acetic anhydride (3.25 mL) is added to the reaction mass dropwise in 15 minutes at 30°C.The reaction mass is heated to 80°C and stirred for 2 hours at the same temperature. The reaction mass is cooled to 30°C after completion of the reaction and water (250 mL) is added. Dichloromethane (50 mL) is added to the reaction mass and stirred for 15 minutes at 30°C. The layers are separated and the aqueous layer is extracted with dichloromethane (50 mL). The combined organic layer is washed with brine solution and dried over sodium sulphate. The solvent from the organic layer is evaporated to obtain a residue. The obtained residue is evaporated with hexane to obtain the product as crude (solid). The crude product is recrystallized in mixture of dichloromethane and hexane. Yield: 3 g.

**Example 12: Preparation of 7-acetoxynaphtalene (compound of Formula II):** 2-naphthol (100 g), acetone (600 mL) and potassium carbonate (100 g) are charged into a round bottom flask at 28°C and stirred to obtain suspension. Acetic anhydride (100 mL) is added drop wise to the reaction mass at 35°C in 45 minutes under stirring. The reaction mass is heated to 55°C and stirred for 1 hour 30 minutes at the same temperature. Water (500 mL) is added to the reaction mass slowly at 50°C and distilled out the solvent from the reaction mass under vacuum. Water (1000 mL) is added to the reaction mass and stirred for 2 hours at 30°C. The solid product is obtained by filtration and washed with water (500 mL) and dried under suction. The obtained solid product and acetone (100 mL) are charged into a round bottom flask at 30°C and stirred to get a clear solution. Water (1500 mL) is added to the clear solution slowly and stirred for 1 hour at 30°C. The product is obtained by filtration and washed with water (300 mL) and dried under suction. The material is further dried under vacuum at 50°C for 7 hours to obtain the title compound as off- white solid. Yield: 120 g; HPLC purity: 99.7%.

**Example 13: Preparation of 1-chloroacetyl-7-acetoxynaphthalene (compound of Formula III):** Dichloromethane (3500 mL) is charged into a round bottom flask at 25°C and cooled to 0°C. Aluminium chloride (1600 g) is added to the flask containing dichloromethane at 0°C under strring. Chloroacetyl chloride (325 mL) is added drop wise to the reaction mass at 0°C in 40 minutes and further cooled to -10°C. The solution of 7-acetoxynaphthalene (500 g) dissolved dichloromethane (1500 mL) is added slowly at -10°C in 2 hours and maintained for 3 hours 30 minutes at the same temperature. The reaction mass is added to the chilled 2N hydrochloric acid solution and stirred for 15 minutes. The layers are separated and the organic layer is washed with 1% aqueous solution of sodium bicarbonate. The organic layer was evaporated at 45°C to obtain residue and methanol (1000 mL) is charged into residue. The solvent is again distilled and methanol (6000 mL) added and heated to 55°C and maintained for 2 hours at the same temperature. The reaction mass is cooled to 25°C and stirred for 1 hour 30 minutes. The product is obtained by filtration and washed with methanol (300 mL) and dried under suction. The product is further dried under vacuum at 55°C for 3 hours to obtain the title compound. Yield: 378 g; HPLC purity: 99.3%.

**Example 14: Preparation of 1-(2-chloroethyl)-7-hydroxynaphthalene (Compound of Formula IVa):** 1-chloroacetyl-7-acetoxynaphthalene (350 g) and dichloromethane (2450 mL) are charged into a round bottom flask at 25°C. Triethylsilane (715 mL) is added to the reaction mass drop wise at 20°C. The reaction mass is cooled to -10°C and titanium tetrachloride (367 mL) is added drop wise in 1 hour 30 minutes. The reaction mass is maintained for 1 hour 30 minute at 0°C. Diluted hydrochloric acid (350 mL) is added slowly to the reaction mass in 30 minutes and maintained for 1 hour 40 minutes at 25°C. The layers are separated and the organic layer is charged into the flask. The pH of the reaction mass adjusted to 8.0 to 8.5 by using aqueous solution of potassium carbonate (70 g in 1750 ml Water) at 25°C and stirred for 10 minutes. The layers are separated and the organic layer charged into the flask and Methanol (1050 mL) to the flask. Potassium carbonate (186 g) is added to the reaction mass and maintained for overnight at 30°C. The aqueous hydrochloric acid solution (227 mL of concentrated HCl in 1750 mL water) is added slowly to the reaction mass at 25°C and stirred for 40 minutes at 30°C. The layers are separated and the organic layer is distilled at 45°C to obtain residue. Hexane (1750 mL) is added to the residue under stirring and cooled to 0°C and maintained for 2 hours. The product is obtained by filtration and washed with chilled hexane (350 mL) and dried under suction. The product is further dried at 35°C for 3 hours 30 minutes. Yield: 221 g; HPLC purity: 98.58%

**Example 15: Preparation of sodiumdiformylamide:** Sodium methoxide (200 g) and dimethylformamide (1000 mL) are charged into a round bottom flask at 29°C and stirred for 30 minutes. Formamide (310 mL) is added drop-wise to the flask in about 30 minutes and heated the reaction mass to 100 °C and maintained for 5 hours at the same temperature. Distilled out dimethylformamide (400 mL) from the reaction mass and then the reaction mass is cooled to 30°C. The solid product is obtained by filtration and washed with dimethylformamide (200 mL) and dried under suction. The material is further dried under vacuum at 70°C for 6 hours. Yield: 300 g.

**Example 16: Preparation of 1-(N, N-diformylaminoethyl)-7-methoxy naphthalene (compound of Formula VIII):** 1-(2-chloroethyl)-7-hydroxynaphthalene (35 g), potassium carbonate (60.7 g) and methanol (175 mL) are into a round bottom flask at 28°C. The reaction mass is cooled to 15°C. Dimethyl sulphate (39.6 mL) is added slowly in 25 minutes at 15°C and maintained for 2 hours at 25°C. Water (420 mL) and ethyl acetate (175 mL) are charged in the reaction mass and stirred for 30 minutes. The layers are separated and the organic layer is washed with aqueous sodium chloride solution. The organic layer is distilled at 50°C and dimethylsulfoxide (105 mL) is added. The reaction mass is cooled to 25°C and sodium diformylamide (64.3 g) added. Tetrabutylammoniumiodide (0.7 g) is charged to the reaction mass and heated to 90°C and maintained for 2 hours. The reaction mass cooled to 28°C and water (350 mL) and ethyl acetate (175 mL) are added and stirred for 30 minutes. The layers are separated and the organic layer is washed with 10% aqueous sodium chloride solution. The layers are separated and the organic layer is distilled at 50°C and toluene (17.5 mL) is added and again distilled to obtain residue. The toluene (105 mL) is added to the residue and heated to 60°C and stirred to get clear solution. n-Hexane (263 mL) is added drop wise to the solution at 30°C and cooled to 0°C and maintained for 30 minutes. The product is obtained by filtration and washed with n-hexane (35 mL) and dried under suction. The product is dried under vacuum at 45°C for 2 hour. Yield: 31.6 g; HPLC purity: 99.46%

**Example 17: Preparation of Agomelatine:** 1-(N, N-diformylaminoethyl)-7-methoxynaphthalene (50 g) and methanol (250 mL) are charged into a round bottom flask at 28°C and stirred for 20 minutes. Concentrated hydrochloric acid (38.18 mL) is added to the reaction mass and heated to 64°C and maintained for 5 hours at the same temperature. The reaction mass is distilled under vacuum to obtain the solid and toluene (100 mL) is added to the solid and again distilled the solvent to obtain suspension. Water (600 mL) and toluene (100 mL) are added to the solution and stirred for 30 minutes at 28°C. The layers are separated and the aqueous layer is washed with toluene (100 mL). The aqueous layer is charged into a flask and activated carbon (5 g) is added and stirred for 30 minutes at 28°C and filtered through hyflo and washed with water (150 mL). The filtrate is taken in a flask and ethylacetate (375 mL) is added. Potassium carbonate solution (prepared by dissolving 70.98 g in 100 mL water) is added to the above solution and stirred for 5 minutes at 28°C. Acetic anhydride (23.3 mL) is added to the reaction mass at 28°C in 10 minutes. After completion of the reaction the layers are separated and the organic layer is washed with 2 % aqueous solution of sodium chloride. The organic layer is distilled under vacuum and toluene (50 mL) is added and again distilled to obtain residue. Toluene (175 mL) is added to the residue and heated to 60°C and maintained for 30 minutes to obtain clear solution. The clear solution is cooled to 12°C and maintained for 2 hours. The solid product is obtained by filtration and washed with toluene (50 mL) and dried under suction. The material is dried under vacuum at 50°C. Yield: 42.5 g; HPLC purity: 99.87%

## Claims

1. A process for preparing 1-(2-chloroethyl)-7-hydroxynaphthalene, a compound of Formula **IVa** comprising:
a) reacting 2-naphthol with an acetylating agent to provide 7-acetoxynaphthalene, a compound of Formula **II**; 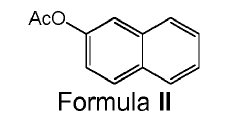
b) reacting the compound of Formula **II** with chloroacetyl chloride to obtain 1-chloroacetyl-7-acetoxynaphthalene, a compound of Formula **III;** 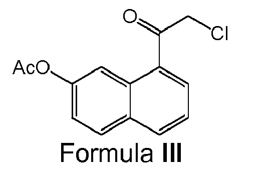
c) reducing the compound of Formula **III** to provide 1-(2-chloroethyl)-7-acetoxynaphthalene, a compound of Formula **IV;** and 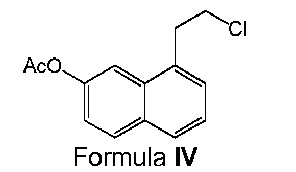
d) deacylating the compound of Formula **IV** to produce 1-(2-chloroethyl)-7-hydroxynaphthalene, a compound of Formula **IVa:**

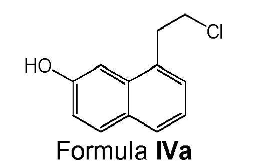

2. A process according to claim 1, wherein the compound of Formula **IV** in step c) is in situ converted into compound of Formula **IVa.**

3. A process for preparing 1-(2-chloroethyl)-7-methoxynaphthalene, a compound of Formula **V** comprising: 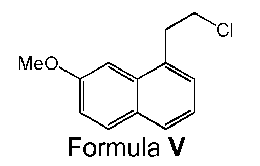
a) reacting 2-naphthol with an acetylating agent to provide 7-acetoxynaphthalene, a compound of Formula **II;**
b) reacting the compound of Formula **II** with chloroacetyl chloride to obtain 1-chloroacetyl-7-acetoxynaphthalene, a compound of Formula **III;**
c) reducing the compound of Formula **III** to provide 1-(2-chloroethyl)-7-acetoxynaphthalene, a compound of Formula **IV;** and
d) hydrolyzing the compound of Formula **IV** followed by methylation with dimethylsulphate to produce the compound of Formula **V**.

4. A process for preparing agomelatine comprising;
a) methylating the compound of Formula **IVa** obtained according to the process of claim 1, with a methylating agent to provide a compound of Formula **V**;
b) reacting the compound of Formula **V** obtained in step a) with an alkali diformylamide salt to provide a compound of Formula **VIII;** 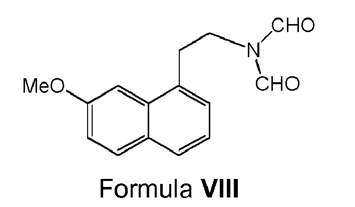
c) hydrolyzing the compound of Formula **VIII** to provide 1-(2-aminoethyl)-7-methoxynaphthalene, a compound of Formula **VII** or its salts; and
d) converting the compound of Formula **VII** or its salts into agomelatine.

5. A process according to claim 4, wherein the compound of Formula **V** obtained in step a) is in situ converted into compound of Formula **VIII.**

6. A process according to claim 4 and 5, wherein an alkali diformylamide used in step b) is sodium diformylamide.

7. A process according to claim 4, 5 and 6, wherein the compound of Formula **VII** obtained in step c) is in situ converted into agomelatine.

8. A process for preparing agomelatine comprising;
a) reacting 1-(2-chloroethyl)-7-methoxynaphthalene, a compound of Formula **V** obtained according to the process of claim 2, with an alkali diformylamide salt to provide 1-(N, N-diformylaminoethyl)-7-methoxynaphthalene, a compound of Formula **VIII;**
b) hydrolyzing the compound of Formula **VIII** to provide a compound of Formula **VII** or its salts; and
c) converting 1-(2-aminoethyl)-7-methoxynaphthalene, a compound of Formula **VII** or its salts into agomelatine.

9. A process for preparing agomelatine comprising;
a) preparing 1-chloroacetyl-7-acetoxynaphthalene from 7-acetoxynaphthalene; and
b) converting 1-chloroacetyl-7-acetoxynaphthalene into aglomelatine.

10. A process for preparing agomelatine comprising;
a) preparing 1-chloroacetyl-7-acetoxynaphthalene from 7-acetoxynaphthalene;
b) reducing 1-chloroacetyl-7-acetoxynaphthalene to produce 1-(2-chloroethyl)-7-acetoxynaphthalene; and
c) converting 1-(2-chloroethyl)-7-acetoxynaphthalene into aglomelatine.

11. 1-chloroacetyl-7-acetoxynaphthalene, a compound of Formula **III.**

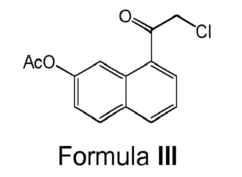

12. 1-(2-chloroethyl)-7-acetoxynaphthalene, a compound of Formula **IV.**

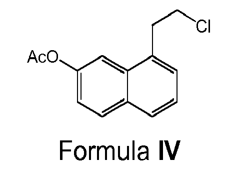

13. 1-(N, N-diformylaminoethyl)-7-methoxy naphthalene, a compound of Formula **VIII.**

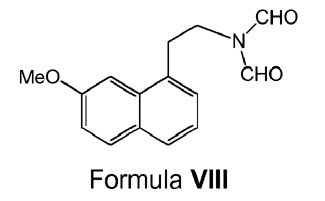

14. Use of compounds of Formula **III, IV** and **VIII** according to the claims 11, 12 or 13 for the preparation of Agomelatine.

15. A pharmaceutical composition comprising agomelatine obtained according to any of the preceding claims and one or more pharmaceutically acceptable carrier.
